# EUROPEAN PATENT SPECIFICATION

(11) **EP 1 935 903 B1**
(45) Date of publication and mention of the grant of the patent: **10.11.2010**
(21) Application number: 06721983.2
(22) Date of filing: 27.02.2006
(51) Int. Cl.: G01N 33/543

(54) **METHOD FOR PRESERVING HUMAN CELL MEMBRANE ANTIGEN AND HUMAN CELL MEMBRANE**
VERFAHREN ZUM KONSERVIEREN VON HUMANEM ZELLMEMBRANANTIGEN
PROCÉDÉ DE PRÉSERVATION DE L'ANTIGÈNE HUMAIN DE LA MEMBRANE CELLULAIRE

(30) Priority: 09.09.2005 CN 200510017117
(43) Date of publication of application: 25.06.2008
(73) Proprietor: Li, Yong, Jilin 130012 (CN)
(72) Inventor: CHEN, Yaguang, Changchun City, Jilin 130012 (CN); TONG, Jun, Changchung City, Jilin 130012 (CN); YANG, Wenchong, Changchun City, Jilin 130012 (CN); LIU, Qinghai, Changchun City, Jilin 130012 (CN); MA, Xueyan, Changchun City, Jilin 130012 (CN); LIN, Hongjie, Changchun City, Jilin 130012 (CN); CHEN, Weijia, Changchun City, Jilin 130012 (CN); SUN, Yuchang, Changchun City, Jilin 130012 (CN); GAO, Dasong, Changchun City, Jilin 130012 (CN); CAI, Hongxia, Changchun City, Jilin 130012 (CN); GU, Dianru, Changchun City, Jilin 130012 (CN); LUAN, Yuxi, Changchun City, Jilin 130012 (CN); LI, Jianbo, Changchun City, Jilin 130012 (CN); WANG, Wenxue, Changchun City, Jilin 130012 (CN)
(74) Representative: Findeisen, Marco
(86) International application number: PCT/CN2006/000283
(87) International publication number: WO 2007/028290

(56) References cited:
- EP-B1- 0 420 186
- CN-A- 1 542 449
- CN-A- 1 595 161
- CN-Y- 2 574 057
- JP-A- 2002 357 594
- US-A- 5 318 914
- OGI M ET AL.: "A novel immunocytochemical staining method that preserves cell membranes: application for demonstrating Ca2+ pump ATPase in the liver" MEDICAL ELECTRON MICROSCOPY, vol. 31, 1998, pages 100-106, XP002520489

## Description

### Technical field

The present invention is in the field of biotechnology, in particular, the present invention relates to a method for preserving cell membrane.

### Background art

The research and detection of human cell membrane antigens and their specific antibodies is one of the most fundamental contents of the experimental research in biological sciences and immunology and clinical detection. For example, the research on HLA antigens and CD markers on human cell membrane is one of the most fundamental basic and applied researches in human immunology. The detection of human ABO and Rh blood group antigens and their antibodies is a routine clinical test. Some biological characteristics such as antigenicity of human cell membrane antigens depend on the survival of the cells. Cell death and dissolution often result in disappearance of antigenicity. For example, if erythrocytes die, the antigenicity of Rh blood group antigens will disappear, and no soluble Rh blood group antigen will be present in the human body. Furthermore, Rh blood group antigens can not be purified, because they are structural proteins of erythrocyte membrane, and thus once shedding from cell membrane, their antigenicity will disappear. Therefore, Rh blood group antibodies must be detected using living erythrocytes. To date, fresh erythrocytes are used as standard antigen in the world in the detection of human ABO blood group antibody, also known as ABO blood group reverse typing. However, the storage time of fresh erythrocytes is short, which is at most 3 to 6 months. Moreover, the antigenicity decreases during the storage. The maintenance of the activity, i.e. antigenicity, of erythrocytes is a problem which has not yet been well solved in life science and immunology. Some of the existing methods for preserving erythrocytes such as cryopreservation (in refrigerator or liquid nitrogen) can hardly freeze a large number of cells, but only preserve specimen in a small amount, e.g. in the case of the preservation of erythrocytes having rare red blood group. The longer the cells are cryopreserved, the weaker their antigenicity is, and the more easily they disappear. As compared with liquid cryopreservation of erythrocytes, lyophilization of erythrocytes theoretically increases the maintenance time of the antigenicity. Even though the research of lyophilization of erythrocytes was reported in a few papers, it has not yet found practical application to date.

As described above, the problems regarding the long-term maintenance of the antigenicity of human cell membrane antigens and the effective application thereof have not yet been solved to date. These problems are represented by the dependency of antigenicity of cell membrane antigen on the survival of the cells. That is to say, the live cells are antigenic, and the dead cells lose their cell membrane antigenicity. Currently, cryopreservation (in refrigerator or liquid nitrogen) is the most commonly used. However, it only maintains the antigenicity of cell membrane in a certain period of time, and it is only suitable for the preservation of especially valuable cell specimens in a small amount.

The present invention specifically relates to a method for preserving human erythrocyte membrane antigen, wherein said method comprising the following steps:
1) preparation of erythrocyte membrane antigen, including the steps of:
   a) centrifuging fresh erythrocytes, discarding the supernatant, and obtaining the packed red cells for the next step;
   b) adding SDS to the packed red cells and keeping at room temperature;
   c) centrifuging, discarding the supernatant, and adding distilled water to the precipitate;
2) preparation of magnetic particles, including the steps of:
   a) dissolution of metal salts: dissolving FeCl₂ and FeCl₃ in distilled water; and
   b) hydrolysis of metal salts: adding sodium hydroxide into the solution of said metal salts with vigorous stirring, thereby obtaining composite metal oxide, namely solution of ferroferric oxide particles; and
3) coating magnetic particles with cell membrane antigen, including the steps of:
   adding erythrocyte membrane antigen obtained from step 1) to the solution of ferroferric oxide particles obtained from step 2); adding polyethylene glycol, incubating, centrifuging, discarding the supernatant and leaving the precipitate; then, adding hypotonic phosphate buffer to the precipitate, thereby obtaining the magnetic particles coated with cell membrane.

The object of the present invention is to completely maintain the antigenicity of cell membrane antigen independent of the survival of the cell. The technical solution used in the invention involves replacing the contents of human cell membrane with ferroferric oxide particles, thus maintaining the antigenicity of the cell membrane antigens. The ferroferric oxide particles coated with cell membrane are magnetic. Under magnetic field, the membrane antigen-antibody complexes resulting from the specific immune reaction of the antigens with the corresponding antibodies in a reaction medium can be isolated and further analyzed by naked eyes or equipments for the immune reaction of the membrane antigen with the antibody through immune aggregation reaction or immune marker detection technology (including ELISA, immunofluorescence analysis, isotope immunoassay, immune luminescent technology, etc.).

In the present invention, living human cells are changed into inactive cells using magnetic particles, but the antigenicity of the cell membrane antigen is maintained. The magnetic particles which can be preserved for a very long time have the antigenicity of the original human cell membrane antigen including that of erythrocyte, platelet, leucocyte, etc. Furthermore, the separation and analysis of particulate antigen-antibody complex in liquid medium are simpler, more efficient, and easier to operate and observe than the conventional centrifugal method.

### Mode of carrying out the invention

The specific procedure of the invention comprises the following steps:
1) preparation of erythrocyte membrane antigen, including the steps of:
   a) centrifuging fresh erythrocytes, and discarding the supernatant. The packed red cells were obtained and treated as follows;
   b) adding SDS to the packed red cells and keeping at room temperature;
   c) centrifuging, discarding the supernatant, and adding distilled water to the precipitate;
2) preparation of magnetic particles, including the steps of:
   a) dissolution of metal salts: dissolving FeCl₂ and FeCl₃ in distilled water; and
   b) hydrolysis of metal salts: adding sodium hydroxide into the solution of said metal salts with vigorous stirring, thereby obtaining composite metal oxide, namely solution of ferroferric oxide particles; and
3) coating the antigen with magnetic particles, including the steps of:
   adding erythrocyte membrane antigen obtained from step 1) to the solution of ferroferric oxide particles obtained from step 2); adding polyethylene glycol, incubating, centrifuging, discarding the supernatant and leaving the precipitate; then, adding hypotonic phosphate buffer to the precipitate, thereby obtaining the magnetic particles coated with cell membrane.

1. The magnetic particles coated with erythrocyte membrane maintain integral antigenicity of blood group antigen.

### 1.1. Preparation of erythrocyte membrane antigen

(1) 9ml of fresh human erythrocyte from 3 persons (3ml per person) were centrifuged at 3000 rpm. Discarding the supernatant and thus obtained 5 ml of packed red cells, wherein 0.5ml was stored, and 4.5ml were treated as follows.
(2) 40ml of 1% SDS were added to the packed red cells, and kept at room temperature for 30 min.
(3) centrifuging at 2000 rpm for 10 min, the supernatant was discarded, and 10ml of distilled water was added to the precipitate.

### 1.2. Preparation of magnetic particles

### 1.2.1 Dissolution of metal salts

Suitable amount of FeCl₂ and FeCl₃ were added to said distilled water and dissolved.

### 1.2.2 Hydrolysis of metal salts

Suitable amount of sodium hydroxide was added to the solution of metal salts as described above with vigorous stirring, thereby producing composite metal oxide, i.e., solution of ferroferric oxide.

### 1.3. Coating magnetic particle with cell membrane

(1) 3ml of solution of ferroferric oxide particles was added to 10 ml of erythrocyte membrane prepared in step 1;
(2) 1ml of polyethylene glycol was added (MW 4000, at a concentration of 50%);
(3) Incubated at 56°C for 1 hr;
(4) Centrifuged at 3000 rpm, the supernatant was discarded and the precipitate was kept;
(5) 10 ml of hypotonic phosphate buffer (PB, pH7.4) was added to the precipitate;
(6) the said solution was distributed;
(7) the ferroferric oxide particles coated with cell membrane was lyophilized, and then was preserved at 4°C.

### 1.4. Detection of the antigenicity of the magnetic particles coated with erythrocyte membrane

Material 1: anti-A, anti-B, anti-D, anti-C, anti-c, anti-E, anti-e blood group monoclonal antibodies

Material 2: the fresh erythrocytes obtained from step 1.1 (1), which are used as control

Material 3: lyophilized magnetic particles coated with erythrocyte membrane

Material 4: phosphate buffer (0.15M, pH 7.4)

Method: hemagglutination test in test tube
(1) the lyophilized magnetic particles coated with erythrocyte membrane was dissolved using 1 ml phosphate buffer;
(2) suspension of fresh erythrocytes at a concentration of 2% using phosphate buffer was prepared;
(3) anti-A, anti-B, anti-D, anti-C, anti-c, anti-E and anti-e blood group monoclonal antibodies was taken out;
(4) as a control, 2% suspension of fresh erythrocytes was reacted with anti-A, anti-B, anti-D, anti-C, anti-c, anti-E and anti-e blood group monoclonal antibodies as control;
(5) 0.10ml of magnetic particles coated with erythrocyte membrane and 0.05ml of different dilutions of anti-A, anti-B, anti-D, anti-C, anti-c, anti-E and anti-e monoclonal anti-blood group antibodies was dropped into a test tube;
(6) Kept at room temperature for 20 min, centrifuged at 1000 rpm for 1 minute, and the results were as follows.

The qualification criterion is shown in the following table.

| Magnetic particles coated with erythrocyte membrane Fresh erythrocyte | | |
|---|---|---|
| Anti-A | 3+ ∼ 2+ | 4+ ∼ 3+ |
| Anti-B | 3+ ∼ 2+ | 4+ ∼ 3+ |
| Anti-D | 2+ ∼ 1+ | 3+ ∼ 2+ |
| Anti-C | 2+ ∼ 1+ | 3+ ∼ 1+ |
| Anti-c | 2+ ∼ 1+ | 3+ ∼ 1+ |
| Anti-E | 2+ ∼ 1+ | 3+ ∼ 1+ |
| Anti-e | 2+ ∼ 1+ | 3+ ∼ 1+ |

### 2. Application

2.1 The detection of complete antibody was exemplified by test of ABO blood group on 96-well U-bottom plate as reverse typing.
(1) Liquid reagents were used directly. Lyophilized reagents need to be thawed. 1 ml of distilled water was added to the lyophilized magnetic particle powder coated with erythrocyte membrane.
(2) 50 µl of serum (plasma) to be tested was dropped into each well of a 96-well U-bottom plate.
(3) 50 µl of corresponding magnetic particles coated with erythrocyte membrane was added per well.
(4) Separated on a magnetic separator for 10 -20 sec.
(5) Shaken on a shaker for 1 min.
(6) the aggregation was observed by naked eyes. The presence of aggregation in a well indicated that an antibody specific for cell membrane antigen coated with magnetic particles was present in the serum, representing a positive reaction. The absence of aggregation in a well indicated that the corresponding specific antibody was not present in the serum, representing a negative reaction.

### 2.2 Detection of incomplete antibody in serum (plasma) samples, taking the detection of RhD blood group anti-D antibody as an example.

Steps (1), (2), (3), (4) and (5) were identical to those of 2.1.
(6) anti-D (monoclonal antibody) was added which is a complete antibody.
(7) After shaking, the presence of aggregation reaction represents a positive reaction, and the absence of aggregation reaction represents a negative reaction.

### Experimental record:

| | |
|---|---|
| Mix each 100 µl of cells A and B + 100 µl of 3% magnetic particles | A majority of cells were not magnetized, and intact cells were present. |
| Mix each 100 µl of cells A and B + 200 µl of 3% magnetic particles | A majority of cells were magnetized, and intact cells were present. |
| Mix each 100 µl of cells A and B + 300 µl of 3% magnetic particles cells | A majority of cells were magnetized, and intact cells were present. |
| Remove the non-magnetiud cells and add 100 µl of 3% magnetic particles | All of the cells were haemolyzed. |

Cells A and B were pooled, respectively, washed once with saline, and then detected.

### Detection:

| | | | | | | |
|---|---|---|---|---|---|---|
| 1. serial dilution of anti-A reagents | 16 fold | 32 fold | 64fold | 128fold | 256fold | 512-fold. |
| Cell A | | | | H(+) | W+ | |
| CeU B | | | | --- | --- | |
| Magnetized cell A | | | | H(+) | W+ | |
| Magnetized cell B | | | | --- | --- | |
| 2. serial dilution of anti-B reagents | 16fold | 32fold | 64fold | 128fold | 256fold | 512fold. |
| Cell A | | | | --- | --- | |
| Cell B | | | | H(+ ) | W+ | |
| Magnetized cell A | | | | --- | --- | |
| Magnetized cell B | | | | H(+) | W+ | |

| | | | | | | |
|---|---|---|---|---|---|---|
| Note: the magnetized cells A and B were aggregated themselves. 300 ml of 3% magnetic particles were added to each 100 ml of panel cells 1, 2, 3, 4, 5, 6, 7 and 8, respectively, and washed twice with physiological saline. | | | | | | |

The specificity of the antigen was detected using a complete antibody.

| | | | | | | | | | | |
|---|---|---|---|---|---|---|---|---|---|---|
| | | | RH | | | Kell | MNS | | Lewis | |
| | D | C | E | c | e | K | M | N | Le^{a} | Le^{b} |
| 1 fresh cell | 4+ | 4+ | - | - | + | | - | 4+ | - | - |
| 1 membrane | | | | | | | | | | |
| antigen magnetic particles | 2+ | 2+ | - | - | + | | - | 2 | - | - |
| 2 fresh cell | 4+ | - | 4+ | 4+ | - | - | 4+ | - | | |
| 2 membrane | | | | | | | | | | |
| antigen magnetic particles | 3+ | - | 2+ | 3+ | - | - | 2+ | - | | |
| 3 fresh cell | 4+ | 2+ | - | - | 2+ | | | | - | - |
| 3 membrane | | | | | | | | | | |
| antigen magnetic particles | 2+ | 2+ | - | - | + | | | | - | - |
| 4 fresh cell | 4+ | - | - | 4+ | 3+ | | | | | |
| 4 membrane | | | | | | | | | | |
| antigen magnetic particles | 3+ | - | - | 3+ | 3+ | | | | | |
| 5 fresh cell | - | 3+ | - | 4+ | 3+ | | | | | |
| 5 membrane | | | | | | | | | | |
| antigen magnetic particles | - | 2+ | - | 3+ | 2+ | | | | | |
| 8 fresh cell | | | | | | 2+ | | | | |
| 8 membrane | | | | | | | | | | |
| antigen magnetic particles | | | | | | 2+ | | | | |

The antigenicity of Lewis was weaker, and no antigenicity was detected on the fresh cell and membrane antigen magnetic particles

The stability of reverse typing reagents:

Reverse typing reagents A and B were boiled in water bath for 0.5h, 1h, 2h, and 2.5h, respectively, and then detected for the antigenicity.

| | | | | | | |
|---|---|---|---|---|---|---|
| membrane antigen magnetic particles | antibody | untreated | treated (h) | | | |
| | | | 0. 5 | 1 | 2 | 2.5 |
| A | -A | 4+ | 4+ | 4+ | 2+ | + |
| membrane antigen magnetic particles | -B | - | - | - | - | - |
| B | -A | - | - | - | - | - |
| membrane antigen magnetic particles | -B | 4+ | 4+ | 4+ | 2+ | 2+ |

Results: after boiled for 1 hr, their antigenicities of A and B membrane antigen magnetic particles were not different from the untreated. However, boiled for 2 hr , the antigenicity decreased.

## Claims

1. A method for preserving human cell membrane antigen, , wherein said human cell is an erythrocyte and said method comprising the following steps:
1) preparation of erythrocyte membrane antigen, including the steps of:
a) centrifuging fresh erythrocytes, discarding the supernatant, and obtaining the packed red cells for the next step;
b) adding SDS to the packed red cells and keeping at room temperature;
c) centrifuging, discarding the supernatant, and adding distilled water to the precipitate;
2) preparation of magnetic particles, including the steps of:
a) dissolution of metal salts: dissolving FeCl₂ and FeCl₃ in distilled water; and
b) hydrolysis of metal salts: adding sodium hydroxide into the solution of said metal salts with vigorous stirring, thereby obtaining composite metal oxide, namely solution of ferroferric oxide particles; and
3) coating magnetic particles with membrane antigen, including the steps of:
adding erythrocyte membrane antigen obtained from step 1) to the solution of ferroferric oxide particles obtained from step 2); adding polyethylene glycol, incubating, centrifuging, discarding the supernatant and leaving the precipitate; then, adding hypotonic phosphate buffer to the precipitate, thereby obtaining the magnetic particles coated with cell membrane antigen.

## Patentansprüche

1. Verfahren zum Konservieren eines Membranantigens einer humanen Zellmembranantigens, wobei die humane Zelle ein Erythrocyt ist und das Verfahren die folgenden Schritte aufweist:
1) Präparieren des Erythrocyten-Membranantigens, einschließlich der folgenden Schritte:
a) Zentrifugieren von frischen Erythrocyten, Verwerfen des Überstandes, und Erhalten der gepackten roten Zellen für den nächsten Schritt;
b) Hinzugeben von SDS zu den gepackten roten Zellen und Halten bei Raumtemperatur;
c) Zentrifugieren, Verwerfen des Überstandes, und Hinzugeben von destilliertem Wasser zu dem Präzipitat;
2) Präparieren von magnetischen Partikeln, einschließlich der folgenden Schritte:
a) Auflösung von Metallsalzen: Auflösen von FeCl₂ und FeCl₃ in destilliertem Wasser;
b) Hydrolyse von Metallsalzen: Hinzugeben von Natriumhydroxid in die Lösung der Metallsalze unter kräftigem Rühren, wobei ein Kompositmetalloxid erhalten wird, nämlich eine Lösung von Ferroferricoxid-Partikeln; und
3) Beschichten der magnetischen Partikel mit Membranantigen, einschließlich der folgenden Schritte:
Hinzugeben des aus Schritt 1) erhaltenen Erythrocyten-Membranantigens zu den aus Schritt 2) erhaltenen Ferroferricoxid-Partikeln; Hinzugeben von Polyethylenglykol, Inkubieren, Zentrifugieren, Verwerfen des Überstandes und Belassen des Präzipitates; anschließend Hinzugeben von hypotonem Phosphatpuffer zu dem Präzipitat, wobei die mit dem Zellmembranantigen beschichteten magnetischen Partikel erhalten werden.

## Revendications

1. Procédé de préservation des antigènes de membrane cellulaire humaine, où ladite cellule humaine est un érythrocyte et ledit procédé comprend les étapes suivantes :
1) la préparation des antigènes de la membrane érythrocytaire, comprenant les étapes consistant à :
a) centrifuger des érythrocytes frais, jeter le surnageant et récupérer les érythrocytes concentrés pour l'étape suivante ;
b) ajouter du SDS aux érythrocytes concentrés et garder à température ambiante ;
c) centrifuger, jeter le surnageant et ajouter de l'eau distillée au précipité ;
2) la préparation des particules magnétiques, comprenant les étapes consistant à :
a) dissoudre des sels métalliques : dissoudre du FeCl₂ et du FeCl₃, dans de l'eau distillée ; et
b) hydrolyser les sels métalliques : ajouter de l'hydroxyde de sodium dans la solution desdits sels métalliques avec une agitation vigoureuse, obtenant de cette manière un oxyde métallique composite, à savoir une solution de particules d'oxyde ferroferrique ; et
3) l'enrobage des particules magnétiques avec les antigènes membranaires, comprenant les étapes consistant à :
ajouter les antigènes de la membrane érythrocytaire obtenus de l'étape 1) à la solution de particules d'oxyde ferroferrique obtenue de l'étape 2) ; ajouter du polyéthylène glycol, incuber, centrifuger, jeter le surnageant et laisser le précipité ; puis, ajouter du tampon phosphate hypotonique au précipité, obtenant de cette manière les particules magnétiques enrobées des antigènes de la membrane cellulaire.
